# EUROPEAN PATENT SPECIFICATION

(11) **EP 4 168 786 B1**
(45) Date of publication and mention of the grant of the patent: **23.07.2025**
(21) Application number: 21736696.2
(22) Date of filing: 10.06.2021
(51) Int. Cl.: G01N 25/04, G01N 33/202

(54) **APPARATUS FOR THE ANALYSIS OF NON-FERROUS METAL ALLOYS AND PARTICULARLY FOR 'S.T.A.' TYPE ANALYSES AND A METHOD OF OPERATION OF SUCH APPARATUS**
VORRICHTUNG ZUR ANALYSE VON NICHTEISENMETALL-LEGIERUNGEN, INSBESONDERE FÜR 'S.T.A' TYPANALYSEN UND VERFAHREN ZUM BETRIEB SOLCH EINER VORRICHTUNG
APPAREIL D'ANALYSE D'ALLIAGES MÉTALLIQUES NON FERREUX ET EN PARTICULIER D'ANALYSES DE TYPE "S.T.A." ET PROCÉDÉ DE FONCTIONNEMENT DUDIT APPAREIL

(30) Priority: 19.06.2020 IT 202000014695
(43) Date of publication of application: 26.04.2023
(73) Proprietor: Duedi S.r.l., 35010 Santa Giustina in Colle (PD) (IT)
(72) Inventor: DABALA', Manuele, 30174 Mestre (Carpenedo) (VE) (IT); YAZDANPANAH, Arshad, 35012 Camposampiero (PD) (IT); DE LISSANDRI, Luca, 35010 Trebaseleghe (PD) (IT); DE LISSANDRI, Mauro, 35010 Santa Giustina in Colle (PD) (IT)
(74) Representative: De Sandre, Emanuele
(86) International application number: PCT/IB2021/055090
(87) International publication number: WO 2021/255587

(56) References cited:
- EP-A1- 1 056 995
- EP-A1- 2 565 615
- EP-B1- 1 056 995
- DE-U1- 9 015 065
- US-A1- 2005 151 306
- GUTIÉRREZ J M ET AL: "Statistical Study to Evaluate the Effect of Processing Variables on Shrinkage Incidence During Solidification of Nodular Cast Irons", METALLURGICAL AND MATERIALS TRANSACTIONS A, SPRINGER US, NEW YORK, vol. 48, no. 4, 30 January 2017 (2017-01-30), pages 1827 - 1840, XP036185831, ISSN: 1073-5623, [retrieved on 20170130], DOI: 10.1007/S11661-017-3969-1

## Description

### DESCRIPTION

The present invention relates to an apparatus for the analysis of non-ferrous metal alloys, particularly for 'S.T.A.' type analyses, a system for the quality control of light alloys comprising said apparatus, as well as a method of use of said apparatus.

The present invention is directed to the realisation of a new predictive model of the behaviour, during solidification, of a non-ferrous alloy and to the development of a system for controlling the quality of a non-ferrous alloy by solidification thermal analysis of the non-ferrous alloy in the molten state, with a time-dependent cooling rate.

The object of the present invention is to develop an apparatus and a method for checking the quality of a liquid non-ferrous metallic material in a few minutes; the method is particularly flexible and usable for a wide range of non-ferrous metallic materials.

The analysis carried out with an apparatus and a method according to the present invention is a thermal analysis of the cooling curve during the solidification of the molten metallic material.

It is known that melting metallic material is one of the most historically popular methods for producing objects and components, in many sectors of industry and beyond [bibl. ref. 1-4].

One of the main problems in the production by melting processes of finished parts is the control of the quality of the metallic material in the molten state.

This control is achieved by analysing the chemical, microstructural and mechanical properties of a certain number of solidified samples.

The weight percentage of the elements of a metal alloy, imperfections and impurities infact can have a great influence on the quality of a casting; therefore, controlling the quality of the melt is the most important step in production in order to obtain final products of acceptable quality [3].

One of the most effective 'in situ' quality controls of a metallic material in the molten state is the thermal analysis of the cooling curve during solidification, whose acronym is STA (Solidification Thermal Analysis) [5,6].

This method has been well developed for cast iron and its alloys in recent decades. The STA method is now used in most iron foundries as the primary check of the quality of the molten metallic material.

In just a few minutes, a STA analysis allows the accurate estimation of different aspects of the molten cast iron, such as the chemical content of carbon, silicon and the carbon equivalent percentage, as well as microstructural data such as the percentage of ferrite-perlite, the presence of micro and macro shrinkage in the metallic material during solidification, the nodule count and the nodularity of the spheroidal cast iron; the STA analysis also allows mechanical predictions to be made with regard to yield strength, tensile strength, hardness and elongation percentage [7-11].

The STA analysis is standardised for cast iron, using dedicated sand-resin cups inside which a thermocouple is fixed [6].

With the STA analysis method, foundries are able to increase productivity, as it is a quick, reliable and cost-effective method compared to other analytical methods.

Thanks to the important advantages of the STA analysis, the number of returns from foundries due to quality problems is significantly reduced, contributing to the increasing diffusion of this analysis method in all cast iron foundries.

It is also known that the use of non-ferrous metallic materials is increasing rapidly due to their excellent mechanical properties and low density compared to other manufacturing alloys [12-14]; in particular, light alloys are now used in various sectors such as aerospace, chemistry, automotive, biomechanics and many other sectors [15].

One of the most critical concerns of the companies making parts from non-ferrous metal materials alloys is the quality of the parts made of light alloy. In addition, scientists are constantly developing new alloys and compounds to meet the growing demand for the use of light alloys in industries.

The quality control of the parts made from a metal alloy is the most important step in production, as without passing specific qualification examinations these parts are not certified for the industrial applications for which they are intended. The first step in the production of light alloys for various industrial uses is the melting and homogenisation of metallic raw materials.

In addition, the demand for recycling the materials used is increasing rapidly worldwide, making a melt quality control system that is simple, flexible, cost-effective and reliable more and more crucial.

The STA solidification thermal analysis is an indispensable method for 'in situ' quality control of molten metallic material prior to any further processing.

In fact, each metal alloy has a unique solidification curve that is like a fingerprint of the alloy; a slight change in the chemical composition of an alloy, or the presence of impurities of intermetallic particles, are parameters that influence the solidification cooling curves [15-21].

These changes can be detected by highly sensitive and accurate apparatuses, including high-precision thermocouples and analogue and digital data loggers. The data obtained from the solidification cooling curves can be analysed for further insights into the material characteristics, such as the percentage of solid fraction, the spacing of the dendritic arms, the coherence point of the dendrites, and the data on mechanical properties as well as the microstructural and chemical composition properties [20-28].

Unlike other material analysis methods that are time-consuming and difficult to implement, the STA analysis is easy to use and can be performed in a couple of minutes to check the quality of the melt directly at the foundry.

In recent decades, the idea of verifying the quality of the melt of non-ferrous metallic materials has been thoroughly investigated by many researchers thanks to its many beneficial effects.

Applying a STA analysis method to non-ferrous metallic materials is more difficult than applying it to cast iron for many reasons, such as the high thermal conductivity, the slight change in the solidification curves as the elements making up an alloy change, and the low melting point of most light alloys.

Over time, researchers have tried to optimise data acquisition methods to overcome difficulties with respect to cast iron.

Extensive researches have been carried out in the area of STA analyses for light alloys, in order to identify the effects of alloying elements and of the impurities on the resulting solidification curves, and to estimate the microstructural and mechanical properties [18, 20, 22, 29, 29-35].

Today's known systems for performing STA analyses on non-ferrous metal alloys are mostly the result of academic researches, and the use of such systems in foundries would require extensive knowledge and technical time. For example, patent document US20050151306A1 [36] describes an apparatus called 'UMSA' designed primarily for a rapid hardening; with such a system, the user generally has limited control over the cooling rate during solidification. Another limitation of the 'UMSA' apparatus is that the position of the thermocouple is not stable and may change with each measurement. Furthermore, as the system is designed to work only with crucibles, it is not possible to measure the temperature of the molten metallic material close to the wall in a safe and stable position. In addition, the system is mainly designed to re-melt the solidified material. The compositional and microstructural characterisation of the sample may differ from the molten material used for melting. For example, the effect of strontium on the microstructure of the aluminium-silicon alloys can be active or passive; this means that although it is possible to detect strontium in the chemical composition of the material with quantum analysis apparatuses, the result of these analyses does not give any information on the modifying effect of strontium on the microstructure of the alloy, and a further addition of strontium may be necessary to achieve a modifying effect on the microstructure of the molten material.

For these reasons, complete remelting the solidified metallic sample causes a change in the material behaviour, making the thermal analysis not sufficiently accurate.

In addition, the alloy preparation prior to STA analysis is time-consuming, making the use of the system limited to development and research purposes.

In patent document US2011054829A1, the thermal analysis apparatus is located in a controlled environment in which a differential thermal analysis system is used for working [37]. In this patent document, there is no control over the cooling rate of the sample and it is not possible to change the cooling rate of the test sample.

The patent document US2012250723A1 focuses on a differential thermal analysis system capable of measuring the temperature difference between cups [38]. The system is designed in such a way that the initial acquisition temperature is fixed and further there is no control over the cooling rate during analysis. The patent document DE 90 15 065 describes an apparatus suitable for the analysis of non-ferrous metal alloys which uses a solidification cup inside which there is defined a containment compartment with a heating means and a thermocouple.

The main problems with most current STA analysis systems can be summarised as follows.

A first problem consists in that, in the currently known STA analysis systems the insertion of the thermocouple for temperature detection is done from the upper part of the crucible (cup) of the molten material; this makes it difficult both pouring the molten metallic material into the cup and positioning the thermocouple, which may be inaccurate as regards the position and the height of the thermocouple tip, due to the removal and the repositioning of the thermocouple with each new test; in this situation, infact, the measurement may not be repeatable enough in order to obtain an accurate prediction of the material characteristics.

In order to insert the thermocouple in the same position for all measurements, it is necessary to check the height of the thermocouple for each test, but this solution requires extensive technical times of realisation.

A second problem with currently known STA analysis systems relates to the protection of the thermocouple; infact, small stainless steel tubes are mainly used to protect the thermocouple from the direct contact with the molten metallic material.

This solution means that, after solidification of the metallic material, the small stainless steel tube remains stuck inside the solidified molten metallic material and has to be replaced each time.

A third problem with some currently known STA analysis systems is related to the use of standard sand cups; with this method it is not possible to control the cooling rate and for different cooling rates to be obtained different cup sizes should be used, i.e., using more material for having a lower cooling rate and having different sized sand cups available.

On the other hand, sand cups, with an internal thermocouple, are themselves a crucible and should be changed each time.

The accuracy of the data acquired is highly correlated to the quality of the sand cups, and if there are small changes in the resin used to bind the sand or in the moisture present in the sand cups, the data may not be sufficiently repeatable. Furthermore, in order to control the quality of the molten metallic material when alloying elements are added, a new cup should be used each time and this might not be an economical choice for foundries.

Another STA analysis system includes the simultaneous use of a plurality of ceramic moulds with different cavity sizes with a pouring channel above these moulds configured to cast the molten metallic material simultaneously into all the cavities of the ceramic moulds.

The problem with this method is that the initial positioning of the thermocouples in each mould is not easy and the repeatability of the measurements is not guaranteed due to a possible difference in the position of the thermocouple and in the size of the moulds; in addition, the molten material required for these types of STA moulds is high and the filling of the entire mould requires special tools and equipment; furthermore, this STA process is time-consuming due to the positioning of the thermocouples and the pre-heating of the ceramic moulds; in addition, the cost of the ceramic moulds is higher than the cost of other crucibles.

A further STA analysis system includes the use of metallic cups of various sizes; these metallic cups have a high thermal conductivity and no control over the solidification rate; this results in a higher cooling rate than other methods but less meaningful data from the solidification curves.

A further problem with currently known STA analysis systems consists in that the temperature measurement near the wall surface is not particularly accurate due to the inaccurate insertion of the thermocouple into the cup and the direct contact of the thermocouple with the molten metallic material; this entails that the position of the thermocouple has to be corrected each time. Furthermore, in such a situation, the thermocouple is not measuring the temperature exactly on the inner surface of the cup wall, but it is measuring the temperature near said wall. Since the dendrite formation begins from the inner surface of the cup wall, the temperature measured near the surface introduces an error into the calculation of the dendritic distance, in jargon 'DAS', Dendrite Arm Spacing, and the calculation of the dendrite coherence point.

All currently known STA apparatuses are mainly intended for development and research purposes, and different cup sizes must be used to achieve different cooling rates. Moreover, with current systems, the cooling rate is not fully controllable and depends mainly on the size of the cup.

The main task of the present invention is to develop an apparatus for the STA type analysis of non-ferrous metal alloys, as well as a method of use of said apparatus, capable of overcoming the above-mentioned limitations of the prior art.

In particular, an object of the invention is to develop an apparatus for the analysis of non-ferrous metal alloys which enables a large amount of information on the quality of the molten material to be obtained quickly and in a repeatable manner.

Another object of the invention is to develop an apparatus for the analysis of non-ferrous metal alloys in which the position of the thermocouples is stable, safe and effective.

Another object of the invention is to develop an apparatus which no longer involves the use of a small metal tube to protect a thermocouple which is lost together with the solidified sample of metal alloy.

Furthermore, an object of the invention is to develop an apparatus for the analysis of non-ferrous metal alloys whose casting cups are reusable.

A further object of the invention is to develop a method of use of a similar apparatus.

The aforementioned task and objects are achieved by an apparatus for the analysis of non-ferrous metal alloys, particularly for 'S.T.A.' type analysis, according to claim 1, as well as by a method of use of said apparatus according to claim 10.

Further characteristics of the apparatus according to claim 1 are described in the dependent claims.

The aforesaid task and objects, together with the advantages that will be mentioned hereinafter, are indicated by the description of two embodiments of the invention, which are given by way of non-limiting example with reference to the attached drawings, where:
- Figure 1 represents a perspective view of an apparatus for the analysis of non-ferrous alloys according to the invention;
- Figure 2 represents a top view of an apparatus according to the invention;
- Figure 3 represents a side view according to the section line III-III of Figure 2;
- Figure 4 represents a perspective view of a detail of the apparatus according to the invention;
- Figure 5 represents a top view of the detail shown in Figure 4;
- Figure 6 represents a side view of the element of Figure 4 according to the section line VI-VI of Figure 5;
- Figure 7 represents a top view of a component of the detail of Figures 2, 3, 4, 5 and 6;
- Figure 8 represents a side sectional view according to the section line VIII-VIII of Figure 7;
- Figure 9 represents a top view of the detail of Figure 5 in an embodiment variant thereof;
- Figure 10 represents a side sectional view according to the section line X-X of Figure 9;
- Figure 11 represents a perspective view of a component of the detail of Figure 9;
- Figure 12 represents a perspective view of an accessory of the apparatus according to the invention;
- Figure 13 represents a longitudinal section of the accessory of Figure 12;
- Figure 14 represents a perspective view of a further accessory of the apparatus according to the invention;
- Figure 15 represents a top view of the accessory of Figure 14;
- Figure 16 represents a block diagram of the method of operation of the apparatus according to the invention;
- Figure 17 represents a subsystem of the method of Figure 16;
- Figure 18 represents an overall diagram of the apparatus according to the invention;
- Figure 19 represents a top view of an apparatus according to the invention in an embodiment variant thereof;
- Figure 20 represents a side view of the apparatus of Figure 19.

With reference to the above-mentioned figures, an apparatus for the analysis of non-ferrous metal alloys, particularly for 'S.T.A.' type analyses, according to the invention, is indicated as a whole with number **10.**

This apparatus **10** comprises:
- a solidification cup **11** inside which a containment compartment **12** is defined; said containment compartment **12** has a truncated-cone shape; alternatively, the containment compartment **12** may have a truncated-pyramidal, or parallelepipedal, or cylindrical, or other concave shape; the containment compartment **12** is intended to accommodate the metallic material in the molten state;
- heating means **13,** configured to heat the solidification cup **11,** positioned so as to surround said containment compartment **12;**
- cooling means **14,** configured to cool the solidification cup **11;** said cooling means **14,** in a first embodiment variant, are positioned so as to surround said containment compartment **12;**
- a protection ferrule **15,** protruding inside the containment compartment **12,** configured to accommodate and cover the end of a thermocouple; said protection ferrule **15** is preferably positioned in the centre of the bottom **12a** of the containment compartment **12;**
- a central thermocouple **16,** inserted into a corresponding through-hole **17** defined at the bottom **11a** of the solidification cup **11,** the end of said central thermocouple **16** being positioned in the protection ferrule **15;**
- ejection means **19** configured to permit the ejection of a body of solidified metallic material from the containment compartment **12,** i.e., after the execution of a test.

The apparatus **10** also comprises locking means **18** configured to stably lock the central thermocouple **16** in its position.

The solidification cup **11** is made, for example, of carbon steel or stainless steel.

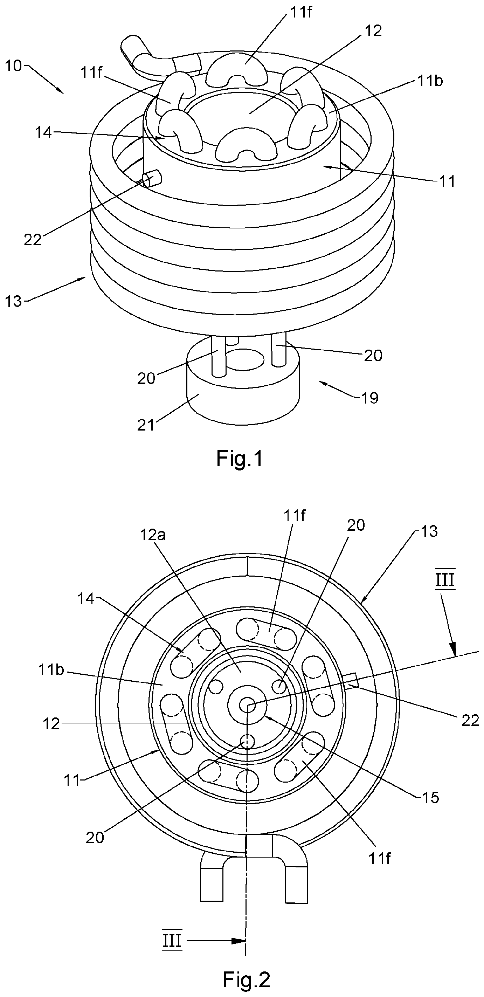

The heating means **13** are, for example, of the induction type, or alternatively of the resistive type; in Figures 1 to 3 such means are schematically shown by a spiral surrounding the solidification cup **11.**

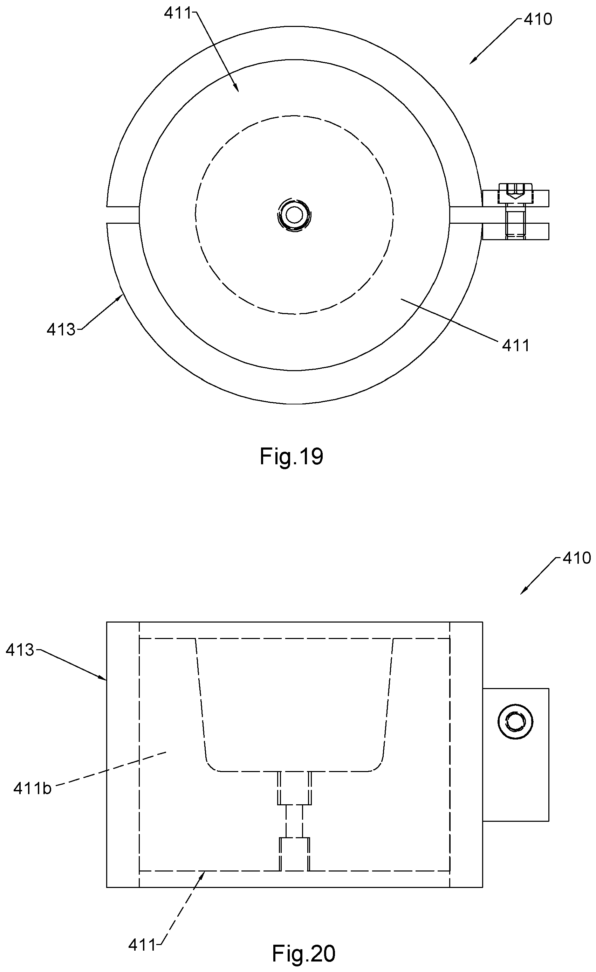

An embodiment variant of the apparatus according to the invention is shown schematically in Figures 19 and 20, and is indicated therein by the number **410.**

In this embodiment variant, the heating means **413** comprise a resistive jacket surrounding the solidification cup **411;** the resistive jacket is in contact with the solidification cup **411.**

The advantage of using induction heating means is that the time required to reach the desired temperature is shorter and the control over the power of the system is more efficient. This offers the possibility of controlling the cooling rate more effectively. On the other hand, the use of induction heating means requires extra equipment such as the circuit and the cooling system for the coils, and the power required for the induction system is also higher than for resistive heating systems. The use of resistive heating is simpler and requires less equipment, but the time required to reach high temperatures is longer than with induction means.

The cooling means **14** comprise, for example, a circulation coil for a cooling fluid, passing through the side wall **11b** of the solidification cup **11.**

Figures 2 to 5 show the longitudinal through-holes **11e** which define the internal sections of the side wall **11b** of the circulation coil through which the cooling fluid passes.

The circulation coil also comprises 'U' fittings between the longitudinal through-holes **11e,** which 'U' fittings are indicated in the figures by the number **11f.**

The cooling means **14** may also comprise auxiliary ventilation means, of a type known per se and not illustrated for simplicity's sake, for example a variable speed fan.

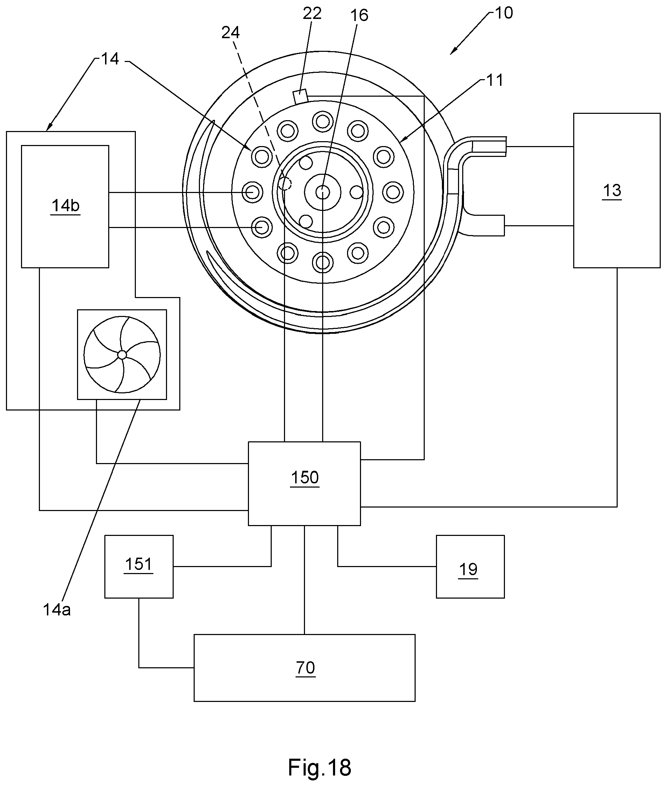

The auxiliary ventilation means are shown in the representative diagram in Figure 18, and indicated there by the number **14a.**

The cooling means **14** therefore comprise at least one chosen from:
- a circulation coil **11e, 11f** for a cooling fluid, passing through the side wall **11b** of said solidification cup **11,**
- and auxiliary ventilation means **14a.**

The cooling means **14** when comprising the refrigerant fluid circulation coil **11e, 11f** obviously also comprise an electric pump for circulating the refrigerant fluid and a heat sink, shown as a whole by box **14b** in Figure 18.

The cooling means **14** may also comprise only one or more auxiliary ventilation means **14a,** in the circuit for lowering the temperature of the cooling fluid, for example a single fan.

For example, but not exclusively, in the embodiment variant of Figures 19 and 20, the solidification cup **411** does not have a circulation coil for a cooling fluid. In Figures 19 and 20, the solidification cup **411** has a side wall **411b** which is full, i.e., it is devoid of the longitudinal through-holes **11e.**

Thus, in such an embodiment variant, the heating means **413** are of the resistive type, with the resistive jacket surrounding the solidification cup **411,** and the cooling means, not illustrated for simplicity's sake, comprise one or more fans directed towards the solidification cup **411.**

As mentioned, said cooling means **14** may therefore comprise both a coil **11e, 11f** for the circulation of a cooling fluid, with an electric pump for circulating the refrigerant fluid and a heat sink **14b,** and auxiliary ventilation means **14a,** consisting for example of a variable speed fan that can be independently activated for simulating the average cooling rate.

The protection ferrule **15** is made of a material with a high heat transfer coefficient.

In the embodiment described herein of the invention, which is intended to be illustrative and not limiting of the invention itself, the protection ferrule **15** comprises a conical body **15a,** for side protection, and an upper cap **15b,** configured to contact the tip of the thermocouple **16.**

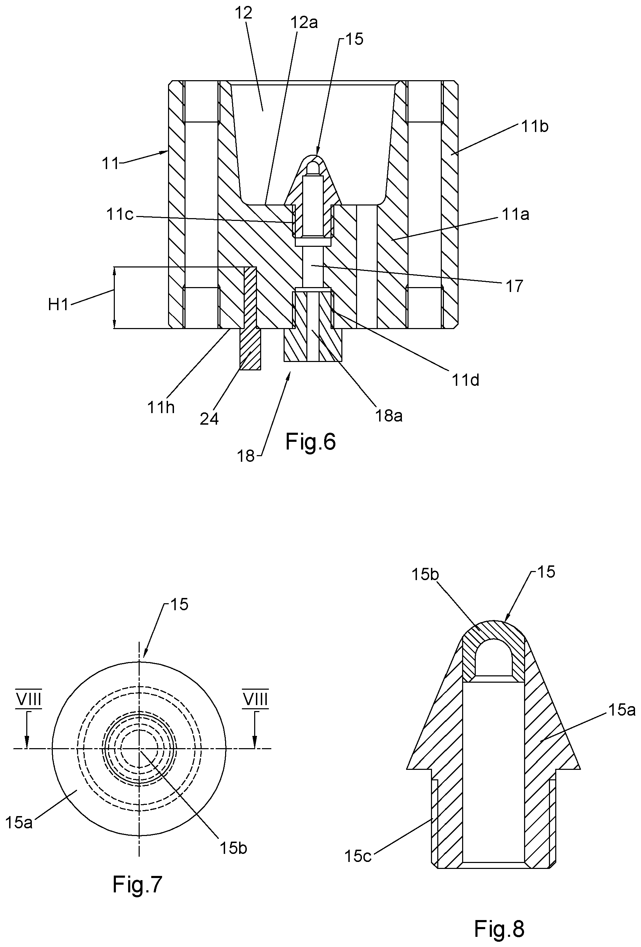

This protection ferrule **15** is clearly visible in Figures 7 and 8.

The conical body **15a** also comprises a threaded shank **15c** for screwing to the bottom **11a** of the solidification cup **11,** at an internally counter-threaded hole **11c.**

The protection ferrule **15** is therefore removable and easily replaceable with a similar one according to specific technical requirements.

The conical body **15a** is made of an alloy with a low heat transfer coefficient, such as for example stainless steel.

The upper cap **15b,** which is in direct contact with the tip of the central thermocouple **16,** is made of a material with high thermal conductivity, e.g., it is made of copper.

Thanks to a similar protection ferrule **15,** a high thermocouple response is achieved even with small temperature variations.

This structure of the protection ferrule **15** makes it possible to avoid the temperature of the tip of the protection ferrule **15** being at a lower temperature than the temperature of the bottom of the solidification cup **11,** a situation which would lead to inaccuracy of the STA analysis and to a thermal shock at the initial moments of the casting of the molten metallic material.

The main advantage of this protection ferrule **15** is given by the high accuracy of the temperature measurement and the fast response to temperature variations.

In order to achieve the most accurate and rapid response of the thermocouple during temperature measurement, the value of the thickness of the upper copper cap **15b** is as small as possible, e.g., comprised between 0.2 millimetres and 3 millimetres.

The central thermocouple **16** is an insulated, shielded stainless steel thermocouple.

The central thermocouple **16** has its tip exposed for best contact with the upper copper cap **15b** of the protection ferrule **15.**

The locking means **18** consists, for example, of a compression fitting comprising an external thread for screwing to a counter-threaded hole **11d** defined externally to the bottom **11a** of the solidification cup **11.**

This compression fitting has an axial through-hole **18a,** clearly visible in Figure 6, inside which a support rod **16a** is stably fixed, e.g., engaged by interference, for the thermocouple **16.**

The ejection means **19** comprise at least one ejection stem **20,** for example three ejection stems **20,** arranged to translate into corresponding through-holes defined on the bottom **11a** of the solidification cup **11,** and supported by a manoeuvring base **21.**

This manoeuvring base **21** is to be understood to be brought into translation towards the bottom **11a,** and vice versa, by a linear actuator, not illustrated for simplicity's sake and to be understood to be of a type known per se, for example mechanical, electrical, pneumatic or hydraulic.

This ejection system allows an easy removal of the solidified metallic material by pushing it out from below.

In an alternative embodiment, not illustrated for simplicity's sake, the solid state specimen ejection means may be configured to extract the same specimen from the top, rather than to push the specimen from below like the ejection means **19** described above.

These means for ejecting the specimen from the top may also be of the mechanical, electrical, pneumatic or hydraulic type.

In a further alternative embodiment, the ejection means are configured to operate both from below and from the top with respect to the solidification cup **11.**

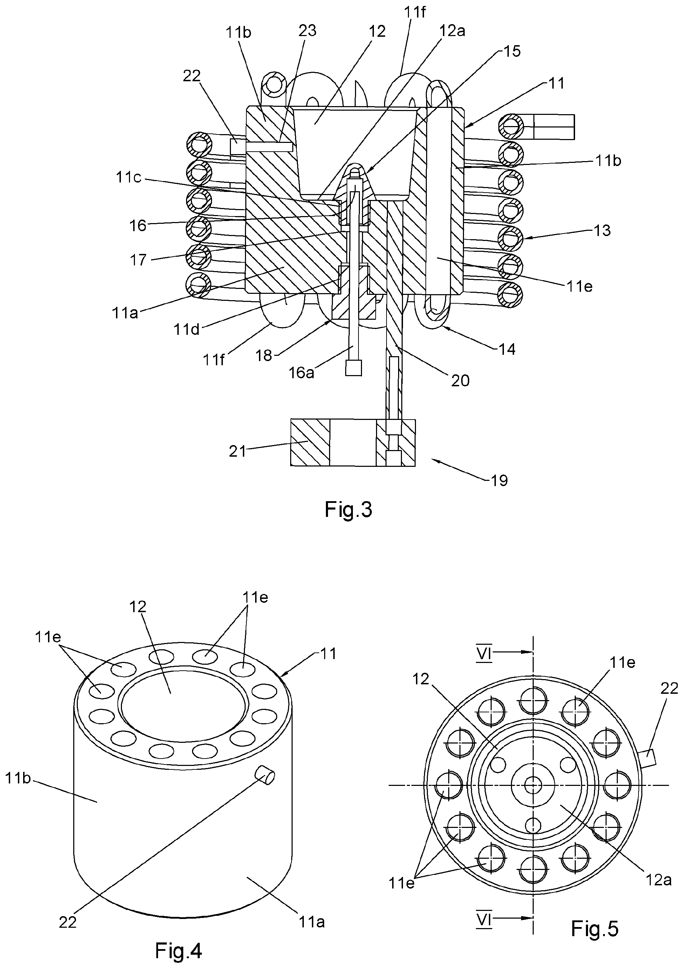

The apparatus **10** also comprises one or more side thermocouples **22,** for example a side thermocouple **22,** clearly visible in Figures 3, 4 and 5.

A side thermocouple **22** is inserted into a corresponding seat **23** defined in the side wall **11b** of the solidification cup **11,** so that the tip of the side thermocouple **22** is close to the inner surface of the containment compartment **12.**

Preferably, but not exclusively, the tip of the side thermocouple **22** is positioned, within the side wall **11b,** at a distance from the inner surface of the containment compartment **12** comprised between 0.1 millimetre and 1 millimetre, these values included.

The solidification cup **11** may have a plurality of seats **23** for a side thermocouple **22,** defined at different heights of the side wall **11b,** available to be occupied by respective side thermocouples **22** depending on the contingent technical requirements.

The side thermocouples **22** are stably locked inside the respective seats, so that their position does not change and the respective detections are therefore reliably repeatable.

The side thermocouples **22** are in contact with at least one inner wall of the respective seats **23** which contain them.

The apparatus **10** further comprises a thermocouple of the cup **24** for measuring the temperature of the solidification cup **11;** for example, said thermocouple of the cup **24** is inserted into a corresponding seat in the bottom **11a,** in contact with at least one inner wall of the seat itself containing it.

In particular, this thermocouple of the cup **24** is positioned with its detecting end close to the centre of the solidification cup **11** in order to make precise temperature measurements.

Preferably, but not exclusively, the thermocouple tip of the cup **24** is positioned at a distance H1, as shown in Figure 6, from the lower surface **11h** of the solidification cup **11** which is half the distance between the same lower surface **11h** and the surface of the bottom **12a** of the containment compartment **12,** as clearly shown in Figure 6.

The apparatus **10** comprises an electronic control unit, schematically shown in Figure 18 and referred to therein with number **150,** configured to collect and process the signals coming from the central thermocouple **16** and from the thermocouple of the cup **24,** as well as from any other thermocouples that may be present, e.g., side thermocouples **22.**

The electronic control unit **150** is configured to command and control the heating means **13** and the cooling means **14,** e.g., by means of corresponding inverters and dedicated micro-controllers.

In particular, the electronic control unit **150** is configured to switch on and off said heating means **13** and said cooling means **14,** and to receive and transmit signals from said central thermocouple **16** and from said thermocouple of the cup **24,** as well as from one or more side thermocouples **22,** if any.

The electronic control unit **150** is in turn connected to an electronic device **70,** for example a personal computer, or a server, or a tablet, or a smartphone, or another similar and equivalent apparatus, to be understood of a known type. The electronic control unit **150** is in turn controlled by the electronic device **70** via a special computer program or a special application ("app").

The electronic control unit **150** collects the signals from the thermocouples **16, 24** and **22** and sends them to the electronic device **70,** where the data are stored and organised.

The electronic control unit **150** comprises for example an analogue-to-digital converter **151,** in jargon 'data logger', for data acquisition.

Said analogue-to-digital converter **151** is schematically shown in Figure 18; said analogue-to-digital converter **151** may be integrated in a containment body of the electronic control unit **150,** or may be external thereto, and in any case it is intended to be part of said electronic control unit **150.**

The containment compartment **12** of the solidification cup **11** is used as a crucible for pouring molten metallic material.

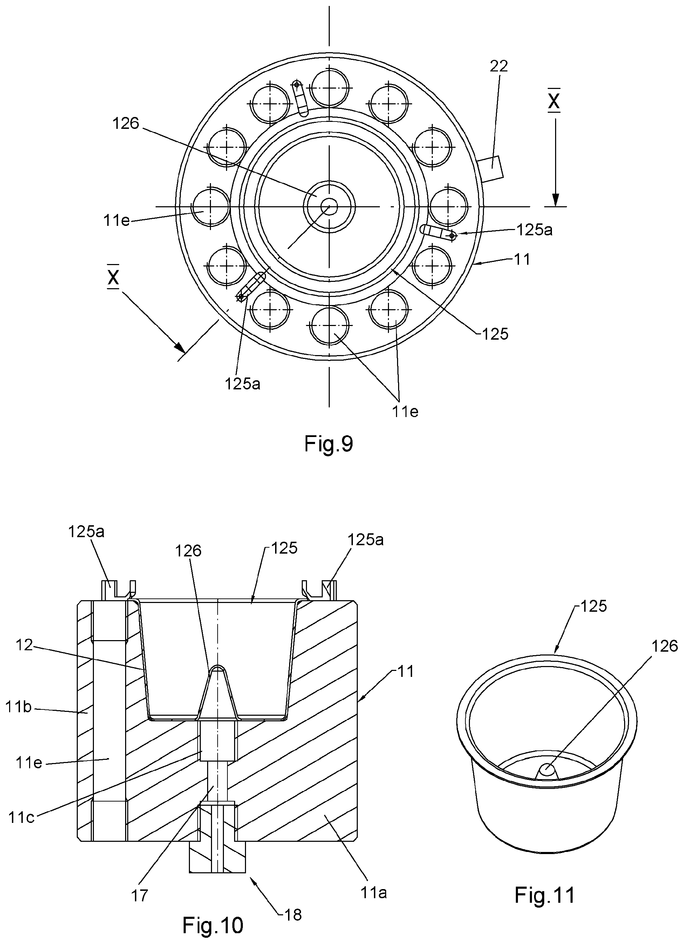

In an alternative embodiment depicted in Figures 9, 10 and 11, the apparatus **10** comprises a removable glass-shaped crucible **125** to be inserted into the containment compartment **12,** and removably inserted into the same containment compartment **12.**

This removable glass-shaped crucible **125** has a truncated-cone shape, or truncated-pyramidal, or parallelepiped, or cylindrical shape, in general a concave shape, depending on the needs and technical requirements.

Said removable glass-shaped crucible **125** has a central relief **126** configured to accommodate the central thermocouple **16.**

The central relief **126** is, of course, hollow.

Said removable glass-shaped crucible **125** comprises a metal body, e.g., of steel, having walls with reduced thickness, e.g., comprised between 0.2 and 3 millimetres.

The apparatus **10** according to the invention is configured in such a way that it can be operated with both systems, i.e., both by using the containment compartment **12** as a crucible for pouring the molten metallic material for STA analysis, and with the removable glass-shaped crucible **125** positioned within the containment compartment **12.**

The surface of the containment compartment **12,** when used as a crucible, has a protective coating to prevent the molten material from sticking to the wall of the containment compartment **12** itself.

When using the removable glass-shaped crucible **125** positioned inside the containment compartment **12,** the protection ferrule **15** must be removed and the position of the central thermocouple **16** calibrated so that this central thermocouple **16** is in contact with the surface of the central relief **126.**

The shape of the central relief **126** is also truncated-conical.

Alternatively, the shape of the central relief **126** may be truncated-pyramidal, or parallelepipedal, or cylindrical, depending on the needs and technical requirements.

In general, this central relief **126** has the same shape as the protection ferrule **15.**

This central relief **126** is essentially technically and functionally equivalent to the protection ferrule **15.**

The advantage of using a removable crucible **125** consists in that the solidified metallic material and the removable crucible **125** are removed at the end of the STA analysis.

Furthermore, the solidification cup **11** has a containment compartment **12** having a fixed geometry, whereas the removable crucible **125** may be made and chosen from a plurality of removable crucibles having different geometries among themselves, for example different in the inclination of the side wall and in the inclination of the wall of the central relief **126,** which, as explained above, may be truncated-conical, or truncated-pyramidal, or parallelepipedal, or cylindrical.

The removable glass-shaped crucible **125** can be made, for example, by moulding a metal sheet.

A further advantage of using removable glass-shaped crucibles **125** consists in that no protective coating of any kind is required on the surface of the containment compartment **12,** since a removable glass-shaped crucible **125** prevents the molten metallic material from coming into contact with this surface, which therefore does not require any auxiliary protection.

In case removable glass-shaped crucibles **125** are used, in the upper part of the solidification cup **11** there is a plurality of anti-extraction elements **125a,** for example three, configured to hold the removable glass-shaped crucible **125** in a stable position within the containment compartment **12.**

Such anti-extraction elements **125a** may be, for example, rotatable arms hinged to the upper face of the solidification cup **11** and adapted to be rotated from a disengagement position to a locking position in which they are arranged above the upper edge of the removable glass-shaped crucible **125,** thereby preventing the same removable crucible **125** from distancing from the bottom of the containment compartment **12.**

The anti-extraction elements **125a** can be operated manually or through pneumatic, hydraulic or suction drives.

The containment compartment **12** and the protection ferrule **15** are shaped according to standard geometries of angular clearance and lateral inclination.

The lateral inclination angles of the containment compartment **12** and the protection ferrule **15** depend on the height of the containment compartment **12** and on the shrinkage characteristics of the non-ferrous metallic material used.

The apparatus **10** according to the invention must therefore have at least two thermocouples connected to the electronic control unit.

A first thermocouple, the thermocouple of the cup **24,** is used for measuring the temperature of the solidification cup **11** itself, and, as mentioned above, is positioned near the centre of the solidification cup **11** to obtain precise temperature measurements.

The second thermocouple required for the STA analysis is the central thermocouple **16** which is in contact with the protection ferrule **15.**

Depending on the method used for the STA analysis and depending on the data required for the analysis, the apparatus **10** may comprise a number of thermocouples higher than two thermocouples.

In order to measure the temperature of the side surface of the containment compartment **12** of the solidification cup **11,** a side thermocouple **22** can be fixed in a dedicated position at a desired height.

The solidification cup **11** is configured in such a way that the temperature measurement of the side surface of the containment compartment **12** is accurate based on the geometry of the attachment point.

The tip of a side thermocouple **22** is inserted into its seat **23** in the side wall **11b** either directly, or through an intermediate insert.

This intermediate insert can be of the copper pressure type, so that the heat transfer is high and the thermocouple has a rapid response to temperature changes.

This intermediate insert may also be of another type, e.g., of another highly conductive metallic material.

If a removable glass-shaped crucible **125** is used for the STA analysis, the upper cap **15b** is removed and the central thermocouple **16** is brought into direct contact with the removable crucible **125.** The connection between the thermocouple and the removable crucible **125** must be calibrated so as to have the best contact with the crucible at the desired height.

The measurement of the temperature by means of a side thermocouple placed near the side surface of the containment compartment **12,** at the same height with respect to the central thermocouple **16,** and the measurement of the temperature difference between such side and central thermocouple **16,** provide much important information on the coherence of the dendrite and the spacing dimensions of the secondary dendritic arms, using Newtonian or Fourier series developments [27].

With this information, it is possible to predict the shrinkage percentage of the non-ferrous metallic material at both microscopic and macroscopic level, as well as the micro-segregation of the alloying elements during solidification.

The present invention makes it possible to measure the temperature of the side surface of the containment compartment **12** in such a way that data acquisition is reproducible, and in such a way that it is very easy to position the thermocouple at the desired height for all STA analyses.

Another advantage of the invention is that the side thermocouples **22** are positioned, in the side wall **11b,** very close to the side surface of the containment compartment **12,** and the distance between the side thermocouple **22** and the central thermocouple **16** is fixed for all data acquisitions.

This means that the electronic control unit can analyse the data accurately through a software related to data analysis.

In addition, there is no need for covering elements for the side thermocouple **22** as it is never in direct contact with the molten metallic material.

The ejection means **19** eject the solidified sample by pushing it from the bottom. With this system, the side walls of the solidified sample are not touched and can be used for other precision measurements of the shrinkage of the non-ferrous metallic material.

Since most non-ferrous metallic materials such as aluminium, copper, titanium, and others have a high heat transfer coefficient, at high temperature there is a sharp drop in the initial temperature of the molten metallic material immediately after the molten metallic material itself has been poured into the containment compartment **12.**

A too low initial temperature means that the data on the initial moments of solidification cannot be acquired.

Since most users avoid using ladles made of refractory material because of the risk of breakage and consequent contamination of the molten metallic material, a ladle **50** for transporting molten metallic material from a starting vessel to the solidification cup **11** is also an object of the present invention.

Said ladle **50** comprises a container having at least two layers, configured to reduce heat losses during the transfer of the molten metallic material.

This double-layer container is advantageously, but not exclusively, made of metal.

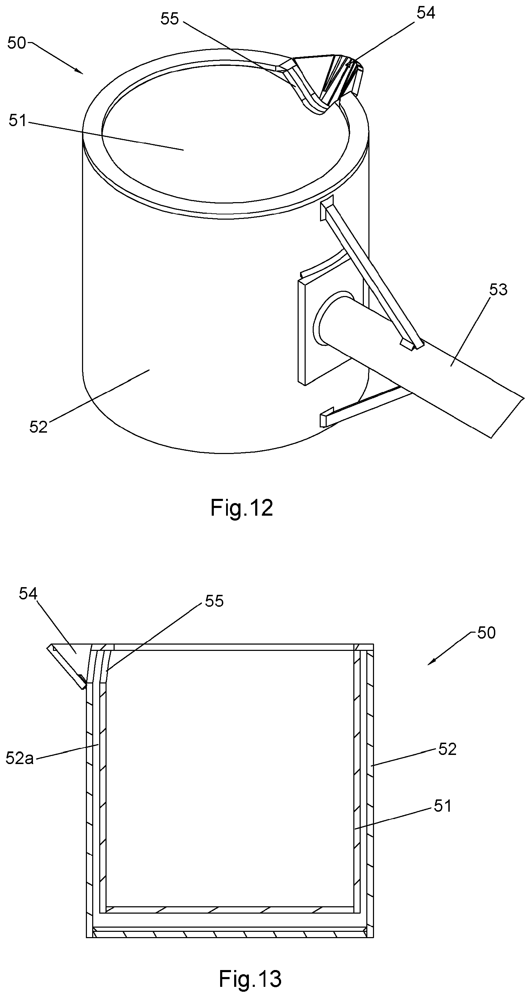

In particular, the ladle **50** is represented in an exemplary embodiment thereof in Figures 12 and 13.

Said ladle **50** comprises an internal container **51** covered with an external structural jacket **52.**

A thermal insulation gap **52a** is defined between the container **51** and the structural jacket **52.**

This gap **52a** is empty.

Alternatively, this gap **52a,** instead of being empty, is intended to be capable of being filled with thermally insulating material, such as for example refractory material, or rock wool, or other similar and technically equivalent thermally insulating material.

The structural jacket **52** has a sleeve **53** for fixing a transport handle not illustrated for simplicity's sake.

The structural jacket **52** has a spout **54,** at which the container **51** has a passage opening **55** configured to favour the passage of molten metallic material.

The entire inner surface of the container **51** is coated with a suitable non-wettable coating to protect the container **51** itself and the melt from contamination by iron and other elements.

In order to be sure of the temperature of the molten metallic material prior to the pouring operation from the ladle **50** to the solidification cup **11,** a thermocouple may be attached to the container **51,** for example through the sleeve **53,** in order to reduce the risk of low melting temperature prior to pouring the molten metallic material into the containment compartment **12.**

With the aid of the ladle **50,** the heat loss of the molten metallic material during the transport of the molten metallic material itself from its vessel to the solidification cup **11** is considerably reduced.

The apparatus **10** according to the invention also comprises a device **60** for measuring the shrinkage percentage of the solidified samples.

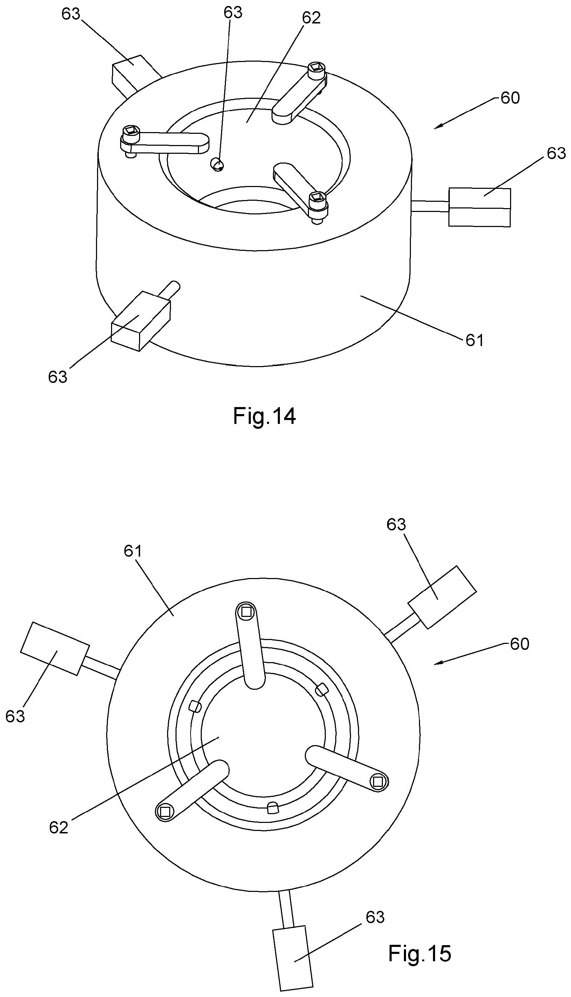

A similar measuring device **60** is shown in Figures 14 and 15.

Such a measuring device **60** comprises a supporting body **61** having a containment compartment **62** shaped to accommodate a solidified sample.

The measuring device **60** comprises at least three contraction sensors **63.**

These contraction sensors **63** consist of manual, mechanical or electronic gauges of commercially known type.

A method of operation of the above-described apparatus **10** is also an object of the invention.

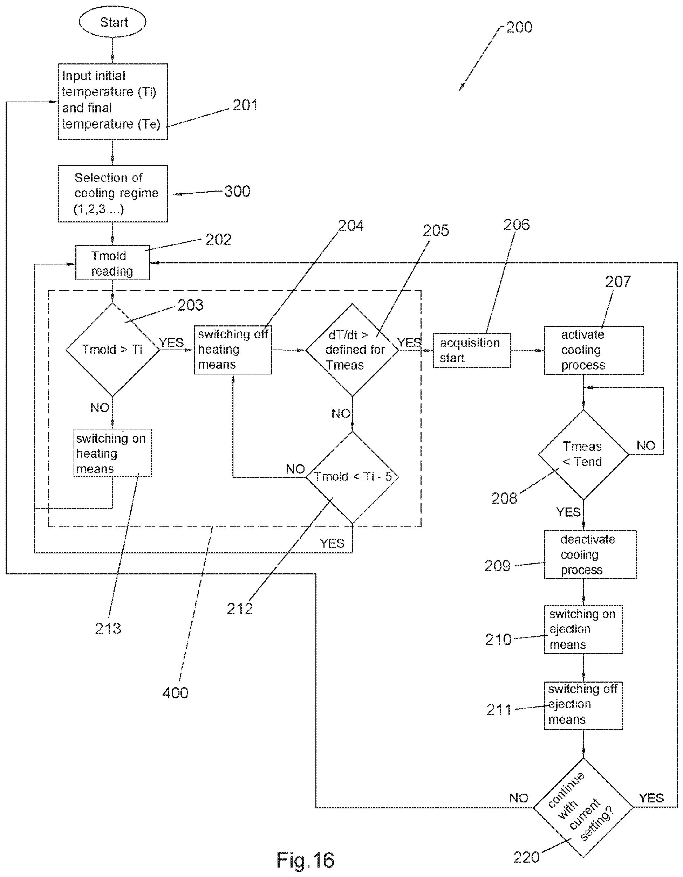

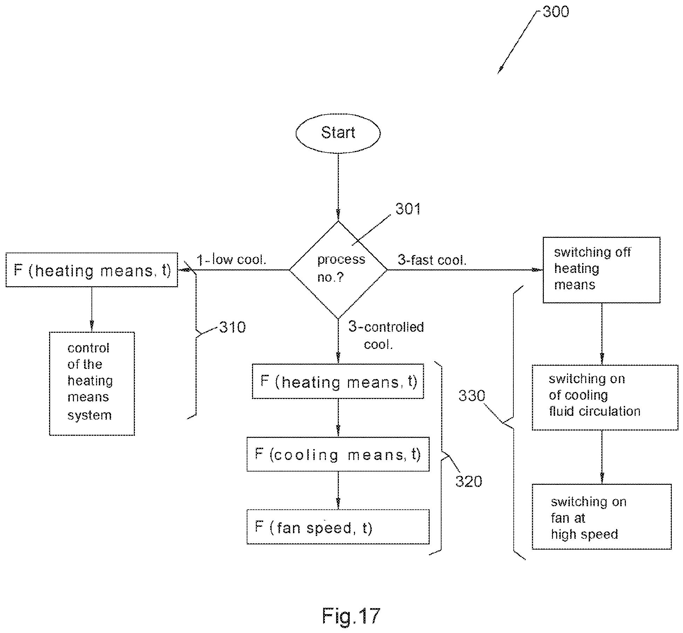

This method of operation comprises an innovative heating and cooling system of the solidification cup **11** and an analysis of the data acquired by the electronic control unit **150** through a software; the software is loaded into the electronic device **70,** for example but not exclusively into a Personal Computer. An example block diagram of this method of operation and data acquisition is shown in Figures 16 and 17.

This method of operation is based on the concept of controlling the cooling rate of the molten metallic material present in the containment compartment **12** by controlling the cooling means **14** and the heating means **13,** by means of micro-controllers, relays and a software, on the basis of two or more variables considered individually or in combination.

The control unit **150** may also comprise micro-controllers and relays for activating and switching off the heating means **13** and the cooling means **14** described above, as well as for connecting the thermocouples **16, 24** and possibly **22.**

All the elements and components configured to activate and deactivate the thermocouples **16, 24** and possibly **22,** the heating means **13** or **413,** the cooling means **14,** the latter possibly comprising a pump for circulating the cooling fluid, and possibly comprising the ventilation means **14a,** and of the analogue-to-digital converter (Data Logger) **151,** as well as all related control or data acquisition apparatuses are located, for example, in a box-like body defining the control unit **150** (with micro-controller and data logger, or any other possible thing such as transistor relays, etc...).

The term 'box-like body' is used to mean any containment element.

It is also to be understood that the components listed above may be present not grouped within a single box-like body, i.e., positioned independently or in sub-groups.

The control unit **150** is connected to the entire system to provide options for acquiring data and controlling all system parameters.

The control unit **150** is connected, for example, to a Personal Computer **70** to acquire data on the non-ferrous metallic material under analysis.

All further specifications described in the Personal Computer are carried out by dedicated software.

Thus, the control unit **150** is interposed between the personal computer **70,** or other equivalent electronic device, and the heating means **13** or **413,** the cooling means **14, 14a, 14b,** and the thermocouples **16, 24** and possibly **22.**

In summary, between the system and the personal computer (with dedicated software) there must be the control unit **150,** which is configured to give orders to the system (control and modification) and also to acquire data (analogue-to-digital converter or data logger for analogue-to-digital conversion).

The heating power, with the aid of the software and a dedicated micro-controller, is a function of the time required for analysis, and it can change linearly or exponentially with time.

In addition, the cooling means **14,** through the control of the ventilation rate and through the activation or non-activation of the circulation of the cooling fluid in the coil surrounding the containment compartment **12,** are programmable with the software as a function of the time required for the analysis.

This means that with this combination of software and hardware, the cooling rate of the non-ferrous metallic material is fully controllable.

For example, in order to have very low to low cooling rates, the power of the heating means **13** changes over time with a predefined formulation as a function of the analysis time set in the software.

Again, for example, in order to obtain low to medium cooling rates, the operation of the heating means **13** can be combined with the operation of the variable speed fan of the cooling means **14.**

Furthermore, thanks to the apparatus **10** according to the invention, it is possible to introduce different cooling rates during the different steps of solidification, in order to study more precisely and in depth a specific temperature range of the solidification curve of the non-ferrous metallic material under examination.

For example, the normal cooling rate of aluminium alloys is very high in the range between the temperature of the liquid and the start of dendrite growth, i.e. the so-called 'liquidus temperature', TL or Tliq, beyond which a material is completely liquid. This temperature is the maximum temperature at which the solid crystals can coexist with the melt in thermodynamic equilibrium and, due to the rapid temperature change, it is difficult to detect specific changes in the cooling rate in the first few seconds after starting to cast the molten material into the containment compartment **12.**

Thanks to the apparatus **10** according to the invention, it is possible to reduce the cooling rate to a rate more suitable for studying a said specific range in the solidification curves.

This means that, with the apparatus **10** according to the present invention, the solidification rate of the metallic material in the containment compartment **12** is fully flexible and controllable.

Since the efficiency, in terms of power and time required, of the induction heating is higher than that of the resistive heating, the use of induction heating is preferable, but obviously not exclusive.

With the use of an induction coil, it is also possible to add a heat dissipation system to more efficiently control the middle to high range of the cooling rates. Furthermore, since all cooling curves are acquired with a modifiable cooling condition, the data can be easily compared; this method therefore offers the possibility of various combinations of parameters to control the cooling rate at any time during solidification.

Another important parameter controlling the cooling rate during solidification is the pre-heating temperature Ti of the solidification cup **11.**

In the STA analysis, the range of solidification temperatures that is important for estimating the quality of the molten metallic material is mostly limited to the range between the temperature of the metallic material in the liquid state and the solidification temperature.

In addition, the temperature gradient between the molten metallic material and the solidification cup **11** plays a vital role in the overall cooling rate of the solidification.

For example, the temperature gradient between 700°C and room temperature is higher than the temperature gradient between 700°C and 400°C due to the general Fourier heat transfer law [39].

Thus, the pre-heating temperature Ti of the solidification cup **11** alone plays a crucial role in an accurate and reliable STA analysis.

The temperature value parameter of the solidification cup **11** is indicated by the abbreviation 'Tmold'.

For the reasons mentioned above, in the present invention, the method of operation, and thus the software that controls the electronic control unit **150** and through it the whole apparatus **10,** is configured to apply pre-heating before data acquisition and after reaching a certain predefined temperature.

Another advantage of this method is that the heat transfer of the molten metallic material is very low during the early steps of temperature acquisition.

The pre-heating temperature Ti of the solidification cup **11** depends on the metal alloy under examination, and on the critical temperature range for the STA analysis, which must be determined by means of a series of calibration measurements for each alloy or group of similar alloys in order to find the best combination of pre-heating temperature and cooling rate.

After setting the parameters for an alloy, or a group of alloys with similar solidification characteristics, the operation of the apparatus **10** can be further calibrated during the tests.

The electronic system for process control consists of two or more systems for the continuous control of the variables related to the heating means **13** and to the cooling means **14,** and one or more relays for starting and stopping other functions such as data acquisition and ejection means **19,** which are all connected to the electronic control unit **150** which in turn is connected to a dedicated personal computer.

All thermocouples **16, 22** and **24,** the control systems of the heating **13** and cooling **14** means, the relays and the ejector system, if equipped with non-mechanical systems, are connected to a micro-controller controlled by dedicated software.

In order to preserve the temperature during the casting time, the ladle **50** is first dipped into the molten metallic material to make its temperature similar to the temperature of the molten metallic material itself.

This reduces the temperature loss of the molten metallic material when pouring the molten metallic material itself into the solidification cup **11.**

The method of operation of an apparatus **10** as described above therefore comprises the following operating steps:
- pre-heating said solidification cup **11** until the temperature Tmold of the solidification cup **11,** detected by means of the thermocouple of the cup **24,** reaches a predefined temperature Ti; Ti is established based on the non-ferrous alloy under analysis;
- pouring the non-ferrous alloy into said containment compartment **12** of said solidification cup **11;** the pouring of the non-ferrous alloy may be carried out in the removable crucible **125,** if any;
- controlled cooling of the non-ferrous alloy by said heating means **13** and by said cooling means **14;**
- measurement of temperature variations Tmeas of the non-ferrous alloy by the central thermocouple **16;** the central thermocouple **16** is connected to said electronic control unit **150;**
- stop of the detection of the temperature Tmeas of the non-ferrous alloy when it reaches a pre-established minimum value Te, detected by the central thermocouple **16;**
- ejection of a sample of solidified non-ferrous metallic material from said containment compartment **12.**

The central thermocouple **16** measures a temperature value indicated with 'Tmeas'.

'Tmeas' stands for 'measured temperature'.

This Tmeas is a value that is referred to the non-ferrous alloy that is cast into the containment compartment **12** of the solidification cup **11.**

This Tmeas is obviously the temperature value assumed by the protection ferrule **15,** which is in contact internally with the central thermocouple **16** and externally with the non-ferrous alloy poured into the containment compartment **12** of the solidification cup **11.**

The method also comprises a step of measuring the shrinkage percentage of said sample of solidified non-ferrous metallic material by means of said measuring device **60.**

Preferably and advantageously, the pouring step is carried out with the ladle **50,** said ladle **50** being pre-heated by positioning it in the molten non-ferrous metallic material prior to withdrawal of the molten non-ferrous metallic material itself.

In particular, the method of operation according to the present invention provides that at the beginning of the acquisition, see block **201** in Figure 16, the user establishes the initial values required for data acquisition and sets the initial state of the system.

The initial values to be established, but not limited to these, are:
- the pre-heating temperature Ti of the solidification cup **11,** whose value is acquired by the thermocouple of the cup **24;** this temperature must be reached before starting data acquisition; this parameter depends on the STA experimental calibration carried out for each type of non-ferrous metallic material (block **201** in Figure 16);
- the minimum temperature Te of the non-ferrous alloy at which data acquisition by the central thermocouple **16** is interrupted; see block **201;** therefore, when the central thermocouple **16** measures this minimum temperature Te, data acquisition by the central thermocouple **16** is interrupted.

Another important parameter for the operation of the method is the maximum temperature tolerance Tmold of the solidification cup **11** with respect to the pre-heating temperature Ti, in order to establish the oscillation frequency, i.e., the switching on and off alternation, of the operation of the heating means **13,** again in order to stabilise the temperature of the solidification cup **11** around the value of the pre-heating temperature Ti.

This maximum tolerance, to which the symbol 'x' is assigned, can assume a value comprised between, by way of example, 3°C and 10°C, e.g., 5°C.

This tolerance value is to be considered with both positive sign and negative sign.

This parameter 'x' is important because the pre-heating temperature Ti of the solidification cup **11** must be maintained under stable conditions during the period of waiting time until the non-ferrous alloy in the liquid state is poured into it.

In order to maintain the temperature Tmold of the solidification cup **11** as close as possible to or equal to the pre-heating temperature Ti, the heating means **13** must be either switched on or off.

Switching on the heating means **13** is determined by the Tmold falling below the value Ti - x, e.g., below the value Ti - 5°C.

Switching off the heating means is determined by Tmold being again equal to Ti or greater than Ti by maximum 5°C.

This step of switching on or not switching on the heating means **13** is schematically shown by block **212** in Figure 16.

Another important parameter for the operation of the method according to the invention is the minimum variation of the temperature Tmeas, so-called 'critical derivative', for the activation of data acquisition by the central thermocouple **16,** and possibly by the side thermocouple **22,** if any.

This parameter is indicated in Figure 16 by the symbol 'dT/dt', i.e., 'temperature variation 'dT' with respect to a time range 'dt'.

If the rate of change of Tmeas is greater than a predetermined value of dT/dt, then it means that the pouring of non-ferrous alloy in the molten state into the containment compartment **12** has started, and the central thermocouple **16** is detecting a rapid increase in the temperature Tmeas; this increase is due to the fact that Tmeas is the temperature of the protection ferrule **15,** which temperature of the protection ferrule before casting the non-ferrous alloy obviously has a temperature similar to the temperature Tmold of the solidification cup **11,** whereas after casting it assumes the temperature of the non-ferrous alloy present in the containment compartment **12.**

When dT/dt is greater than a pre-established value, then data acquisition by the central thermocouple **16** begins. This step is indicated in Figure 16 by block **205.**

The value of dT/dt can be chosen, for example, between 15°C per second and 30°C per second, e.g., dT/dt can have a value of 20°C per second.

Another value to be established may be the duration of activation of the ejection system.

Another important parameter to establish before starting the operation of the apparatus **10** is the cooling regime, see block **300** in Figures 16 and 17.

These initial values indicated above are not intended to be exclusive and limiting to the detection and determination of other parameters.

The cooling procedure is the sequence of operations implemented in the main software code.

With such a sequence of operations it is possible to control the cooling rate with a different combination of parameters of the cooling system. For example, the supply power of the heating means **13** is a function of time and may decrease linearly, exponentially or even in different steps in combination with a constant, or linear, or exponential decrease in temperature.

In addition, it is possible to delay the temperature decrease in specific temperature ranges. An example of three different cooling regimes is shown in Figure 17.

The step of choosing a cooling regime is indicated as a whole by the number **300.**

This step of choosing the cooling regime **300** involves the following operations:
- alternatively choosing one of the three pre-established cooling regimes: slow cooling **310,** controlled cooling **320** and fast cooling **330** (see block **301**);
- if slow cooling **310** is activated, then the control unit of the apparatus **10** controls the heating means **13,** according to a preset function of time F1(t), while the cooling means **14** are switched off;
- if the controlled cooling **320** is activated, then the control unit of the apparatus **10** controls the heating means **13,** according to a preset function of time F2(t), and activates the cooling means **14,** according to a corresponding preset function of time F3(t), i.e., both the refrigerant fluid coil and the fan **14a;**
- if fast cooling **330** is activated, then the control unit of the apparatus **10** deactivates the heating means **13** and activates the cooling means **14,** with the fan **14a** activated at high speed.

As discussed above, during a STA analysis with the apparatus **10,** the temperature is measured by at least two thermocouples for each solidification cup **11.**

The first component whose temperature Tmold is measured is the body of the solidification cup **11.**

The thermocouple of the cup **24** is connected to the solidification cup **11** as described above, and the temperature of the solidification cup **11,** indicated by 'Tmold', is measured in order to stabilise the pre-heating temperature Ti of the solidification cup **11** itself and to control the temperature of the solidification cup **11** throughout the process. The other measurements, through the central thermocouple **16** and the side thermocouple **22,** if any, are carried out to perform the actual STA analysis.

More than one thermocouple can be used in different positions and heights in each solidification cup **11** to collect further information on solidification.

'Tmeas' stands for 'measured temperature', and is the symbol used for measuring the temperature of the non-ferrous alloy by the central **16** and possibly side **22** thermocouples for the STA analysis.

This temperature Tmeas is also used to activate the start and the end of temperature data acquisition.

During the activation of the operation of the apparatus **10,** the heating means **13** increase the temperature of the solidification cup **11** up to the pre-established temperature Ti for the acquisition of said data.

The electronic control unit **150** performs the detection of temperature Tmold of the solidification cup **11** through the thermocouple of the cup **24** (see block **202).**

Tmold is compared with preset Ti.

If Tmold is greater than or equal to Ti, block **203,** then the heating means **13** are switched off, block **204.**

If Tmold is lower than Ti, then the heating means **13** are reactivated (block **213).**

The heating means **13** determine a temperature of the solidification cup **11,** Tmold, that is stable around the value Ti before pouring the molten metallic material.

The method of operation according to the invention comprises a step of detecting the temperature variation in time dT/dt by the central thermocouple **16,** the so-called 'critical derivative', which triggers the automatic start of data acquisition by the central thermocouple **16** and the side thermocouple **22** if any (block **205).**

If the critical derivative of the temperature (heating rate) is equal to or greater than a preset value, then the data acquisition process begins, schematically shown by block **206.**

If the critical derivative of the temperature is lower than the preset value, then the temperature Tmold of the solidification cup **11** is verified (block **212).**

The method of operation according to the invention thus comprises a reiteration of instructions **400,** i.e., a waiting cycle **400** configured to maintain the solidification cup **11** at the pre-established temperature Ti until a non-ferrous alloy in the molten state is poured into it.

Said waiting cycle **400** comprises a first step of checking the value of Tmold, block **203,** the step of switching off the heating means **13,** block **204,** a second step of checking the value of Tmold, block **212,** the step of switching on the heating means **13,** block **213,** and the step of assessing the rate of change of the temperature Tmold, block **205.**

The method provides for the pre-heating temperature Tmold to be known before pouring the non-ferrous alloy. As explained, if the temperature of the mould itself drops, the heating means automatically switch on again.

If Tmold is greater than or equal to a value corresponding to Ti lower than a value of x°, where 'x' is the process variable and is for example equal to 5, if Tmold is greater than or equal to Ti - 5°C, then the heating means **13** are switched off (block **204).**

If Tmold is lower than Ti - x°, for example Ti - 5°C, then the heating means switch on again (block **213).**

In the case of box **212** the figure 5°C is, as mentioned, a process variable.

Following the start of the acquisition process, the cooling means **14** are activated or not, depending on the selected cooling regime, block **207.**

During data acquisition, the method of operation of the apparatus **10** includes controlling the temperature of the thermocouples STA, indicated with Tmeas, and in case of detection of temperatures lower than the predefined temperature Te, block **208,** the cooling means **14** are deactivated and data acquisition is interrupted, block **209.**

Subsequently, the ejection means **19** intervene which eject the solidified sample from the solidification cup **11** (see block **210,** start of the ejection means **19,** and block **211,** stop of the ejection means **19).**

After an acquisition has been successfully completed, the method is started again and the parameters on how to start data acquisition are set again.

If the change of some control parameters is necessary, it can be done before starting with a new acquisition, block **201.**

Then, following the stop of the ejection means **19** (block **211),** if the setting of initial values of Ti and Te is continued (block **220),** then the reading of Tmold is resumed (block **202),** relative to a new molten metallic material poured into the solidification cup **11;** if the setting of initial values is discontinued, then new values of Ti and Te are entered, as per block **201.**

The acquired data are used by the electronic control unit **150,** via software, for further investigation and estimation of the various quality parameters of the molten metallic material.

After removal of a solidified specimen from the solidification cup **11,** it is positioned inside the measuring device **60,** for measuring the volumetric contraction of the solidified material.

This contraction measuring device **60** has at least three sensors for measuring the shrinkage percentage of the non-ferrous metallic material and comparing the results with the STA analysis data.

The indicators and detectors used in the contraction measurement matrix are connected to the computer and the data are automatically displayed in the main software.

The software then uses algorithms to recalibrate the general contraction data estimated from STA analyses with the data obtained through the contraction measuring device **60.** The contraction measuring device **60** is first calibrated by measuring at least one precise reference sample.

After calibration of the contraction measuring device **60,** the STA sample is positioned in the containment compartment **62** and the amount of space between the reference sample and the STA samples is measured with connected gauges; the data are sent to the software for further analyses.

The data acquired from different acquisition sources connected to the solidification cup **11** are then analysed with a dedicated thermal analysis software in order to estimate the effect of the addition of different alloying elements and impurities on the mechanical and micro-structural properties of the solidified sample.

The use of the present invention is completely flexible, and makes it possible to simulate the cooling conditions of small-scale industrial parts in order to understand the actual conditions of segregation and shrinkage of the non-ferrous metallic material in industrial products; for example, process engineers can use the apparatus **10** and the method of operation thereof to gain a better understanding of the behaviour during solidification of a metal alloy, for an efficient design of cores and sections of industrial moulds.

It is to be understood that also a system for the 'S.T.A.' type analysis of light alloys, which is characterised by comprising an apparatus **10** for the analysis of non-ferrous metal alloys as described above and a ladle **50** as described above, form an object of the invention.

The method of operation according to the invention is intended to be practicable with said system.

The method of operation according to the invention is to be understood as also being feasible with apparatuses other than the above-described apparatus **10** that are technically similar and equivalent.

It has in practice been established that the invention achieves the intended task and objects.

In particular, with the invention an apparatus **10** has been developed which allows controlling the cooling rate of the molten metallic material inside the solidification cup, during the STA analysis.

This means that the cooling rate is fully controllable and can be used to modulate the temperature, even controlling it within specific pre-established ranges.

Still particularly, with the present invention, a method of operation of said apparatus has been developed, configured to control the cooling rate of a metallic material in the molten state with a different combination of the parameters of the cooling means, by setting the supply power of the heating means **13** as a function of the analysis time, the pre-heating temperature Ti determined by reading the thermocouple of the cup **24** to be reached before starting the data acquisition, and the activation of the cooling means **14** according to a function of the analysis time F3(t).

Still particularly, with the invention an apparatus for the analysis of non-ferrous metal alloys has been developed which enables a large amount of information on the quality of a non-ferrous metal alloy to be obtained quickly and in a repeatable manner.

Furthermore, with the invention an apparatus for the analysis of non-ferrous metal alloys has been developed wherein the position of the thermocouples is stable, safe and effective.

In addition, with the invention an apparatus has been developed which no longer requires the use of a disposable metal tube, protecting a thermocouple, together with the solidified sample of metal alloy.

Furthermore, with the invention an apparatus for the analysis of non-ferrous metal alloys has been developed whose casting cups are reusable.

In practice, the components and materials used, as long as they are compatible with the specific use, as well as the contingent dimensions and shapes, can be any according to the needs and the prior art.

If the characteristics and techniques mentioned in any claim are followed by reference signs, these reference signs are to be intended for the sole purpose of increasing the intelligibility of the claims and, consequently, such reference signs have no limiting effect on the interpretation of each element identified by way of example from these reference signs.

### Bibliographical references:

[1] T.F. Waters, Fundamentals of manufacturing for engineers, UCL Press, London; Bristol, Pa., 1996. http://site.ebrary.com/id/10099113 (accessed March 11, 2020).
[2] E.P. DeGarmo, ed., Materials and processes in manufacturing, 9th ed, Wiley, New York, 2003.
[3] B. Ravi, Metal casting: computer-aided design and analysis, Prentice-Hall, New Delhi, 2006.
[4] J. Mei, T. Rehren, Beijing ke ji da xue, Institute for Archaeo-Metallurgical Studies, eds., Metallurgy and civilisation: Eurasia and beyond, Archetype, London, 2009.
[5] R.C. Mackenzie, Origin and development of differential thermal analysis, Thermochimica Acta. 73 (1984) 307-367. https://doi.org/10.1016/0040-6031 (84)85200-4.
[6] D.M. Stefanescu, Thermal Analysis-Theory and Applications in Metalcasting, Inter Metalcast. 9 (2015) 7-22. https://doi.org/10.1007/BF03355598.
[7] D.M. Stefanescu, S. Katz, Thermodynamic properties of iron-base alloys, ASM Handbook. 15 (1988) 61-70.
[8] G. Strong, Thermal Analysis as a Ductile Iron Molten Metal Processing Evaluation Tool. (Retroactive Coverage), Transactions of the American Foundrymen's Society. 91 (1983) 151-156.
[9] J. Gutiérrez, A. Natxiondo, J. Nieves, A. Zabala, J. Sertucha, Statistical study to evaluate the effect of processing variables on shrinkage incidence during solidification of nodular cast irons, Metallurgical and Materials Transactions A. 48 (2017) 1827-1840.
[10] D. Sparkman, Microstructure by thermal analysis, AFS Transactions. 119 (2011) 11-068.
[11] F. Hasse, K. Stockholm, Principles of Thermal Analysis, (2017).
[12] M. Okayasu, Y. Ohkura, S. Takeuchi, S. Takasu, H. Ohfuji, T. Shiraishi, A study of the mechanical properties of an Al-Si-Cu alloy (ADC12) produced by various casting processes, Materials Science and Engineering: A. 543 (2012) 185-192.
[13] J.G. Kaufman, E.L. Rooy, Aluminum alloy castings: properties, processes, and applications, Asm International, 2004.
[14] Y. Yamamoto, M. Fujita, N. Sakate, K. Ohuchi, S. Hirabara, Method of producing a light alloy product, Google Patents, 1998.
[15] R. Nunes, J. Adams, Properties and selection: nonferrous alloys and special-purpose materials, American Society for Metals. 2 (1990).
[16] S. Shabestari, M. Malekan, Assessment of the effect of grain refinement on the solidification characteristics of 319 aluminum alloy using thermal analysis, Journal of Alloys and Compounds. 492 (2010) 134-142.
[17] S. Farahany, A. Ourdjini, M. Idrsi, S. Shabestari, Evaluation of the effect of Bi, Sb, Sr and cooling condition on eutectic phases in an Al-Si-Cu alloy (ADC12) by in situ thermal analysis, Thermochimica Acta. 559 (2013) 59-68.
[18] X. Chen, H. Geng, Y. Li, Study on the eutectic modification level of Al-7Si Alloy by computer aided recognition of thermal analysis cooling curves, Materials Science and Engineering: A. 419 (2006) 283-289.
[19] M. Ode, S.G. Kim, W.T. Kim, T. Suzuki, Numerical prediction of the secondary dendrite arm spacing using a phase-field model, ISIJ International. 41 (2001) 345-349.
[20] V. Hosseini, S. Shabestari, R. Gholizadeh, Study on the effect of cooling rate on the solidification parameters, microstructure, and mechanical properties of LM13 alloy using cooling curve thermal analysis technique, Materials & Design. 50 (2013) 7-14.
[21] A. Canales, J. Talamantes-Silva, D. Gloria, S. Valtierra, R. Colás, Thermal analysis during solidification of cast Al-Si alloys, Thermochimica Acta. 510 (2010) 82-87.
[22] L. Zhang, B. Zhou, Z. Zhan, Y. Jia, S. Shan, B. Zhang, W. Wang, Mechanical properties of cast A356 alloy, solidified at cooling rates enhanced by phase transition of a cooling medium, Materials Science and Engineering: A. 448 (2007) 361-365.
[23] L. Heusler, W. Schneider, Influence of alloying elements on the thermal analysis results of Al-Si cast alloys, Journal of Light Metals. 2 (2002) 17-26.
[24] W.S. Ebhota, T.-C. Jen, Effects of modification techniques on mechanical properties of Al-Si cast alloys, Aluminium Alloys-Recent Trends in Processing, Characterization, Mechanical Behavior and Applications (2017) 59-80.
[25] A. Toenjes, A. von Hehl, A fast method for predicting the mechanical properties of precipitation-hardenable aluminum alloys, Metals. 9 (2019) 147.
[26] D. Ferdian, J. Lacaze, I. Lizarralde, A. Niklas, A.I. Fernandez-Calvo, Study of the effect of cooling rate on eutectic modification in A356 aluminium alloys, in: Materials Science Forum, Trans Tech Publ, 2013: pp. 130-134.
[27] I.V. Gómez, E.V. Viteri, J. Montero, M. Djurdjevic, G. Huber, The Determination of Dendrite Coherency Point Characteristics Using Three New Methods for Aluminum Alloys, Applied Sciences. 8 (2018) 1236.
[28] A. Niklas, U. Abaunza, A.I. Fernandez-Calvo, J. Lacaze, R. Suarez, Thermal analysis as a microstructure prediction tool for A356 aluminium parts solidified under various cooling conditions, (2011).
[29] S. Liang, R. Chen, J. Blandin, M. Suery, E. Han, Thermal analysis and solidification pathways of Mg-Al-Ca system alloys, Materials Science and Engineering: A. 480 (2008) 365-372.
[30] F. Yavari, S. Shabestari, Effect of cooling rate and Al content on solidification characteristics of AZ magnesium alloys using cooling curve thermal analysis, Journal of Thermal Analysis and Calorimetry. 129 (2017) 655-662.
[31] A. Luo, Understanding the solidification of magnesium alloys, in: Proceedings of the Third International Magnesium Conference, 1996: pp. 449-464.
[32] L. Dobrzański, M. Król, T. Tański, R. Maniara, Effect of cooling rate on the solidification behaviour of magnesium alloys, Archives of Computational Materials Science and Surface Engineering. 1 (2009) 21-24.
[33] S. Farahany, H.R. Bakhsheshi-Rad, M.H. Idris, M.R.A. Kadir, A.F. Lotfabadi, A. Ourdjini, In-situ thermal analysis and macroscopical characterization of Mg-xCa and Mg-0.5 Ca-xZn alloy systems, Thermochimica Acta. 527 (2012) 180-189.
[34] H. Jafari, M.H. Idris, A. Ourdjini, S. Farahany, In situ melting and solidification assessment of AZ91D granules by computer-aided thermal analysis during investment casting process, Materials & Design. 50 (2013) 181-190.
[35] J. Medved, P. Mrvar, Thermal analysis of the Mg-Al alloys, in: Materials Science Forum, Trans Tech Publ, 2006: pp. 603-608.
[36] M. Kasprzak, W.J. Kasprzak, W.T. Kierkus, J.H. Sokolowski, Method and apparatus for universal metallurgical simulation and analysis, US2005151306A1, 2005.
[37] K. Yamada, Thermal analysis apparatus, US2011054829A1, 2011.
[38] J. Blumm, System And Method For Thermal Analysis, US2012250723A1, 2012.
[39] T.L. Bergman, F.P. Incropera, eds., Fundamentals of heat and mass transfer, 7th ed, Wiley, Hoboken, NJ, 2011.

## Claims

1. Apparatus (10) for the analysis of non-ferrous metal alloys, particularly for 'S.T.A.' type analyses, comprising:
- a solidification cup (11) inside which a containment compartment (12), intended to accommodate a metallic material in the molten state, is defined;
- heating means (13), configured to heat said solidification cup (11), positioned so as to surround said containment compartment (12);
- cooling means (14), configured to cool the solidification cup (11);
- a protection ferrule (15), protruding inside said containment compartment (12), configured to accommodate and cover the end of a thermocouple;
- a central thermocouple (16), inserted into a corresponding through-hole (17) defined at the bottom (11a) of said solidification cup (11), the end of said central thermocouple (16) being positioned in said protection ferrule (15);
- locking means (18) configured to stably lock said central thermocouple (16) in its position;
- ejection means (19) configured to permit the ejection of a body of solidified metallic material away from said containment compartment (12);
- a thermocouple of the cup (24) for measuring the temperature of said solidification cup (11);
- an electronic control unit (150) configured to switch on and off said heating means (13) and said cooling means (14), and to receive and transmit signals from said central thermocouple (16) and from said thermocouple of the cup (24), **characterised in that** it comprises;
- a device (60) for measuring the shrinkage percentage of the solidified samples.

2. Apparatus (10) according to claim 1, **characterised in that** it comprises a removable glass-shaped crucible (125) shaped to be inserted into the containment compartment (12), and removably inserted into the same containment compartment (12), said removable glass-shaped crucible (125) having a central relief (126) configured to accommodate said central thermocouple (16).

3. Apparatus (10) according to claim 1, **characterised in that** said heating means (13) are of the induction type, or alternatively of the resistive type (413).

4. Apparatus (10) according to one or more of the preceding claims, **characterised in that** said cooling means (14) comprise at least one chosen from:
- a circulation coil (11e, 11f) for a cooling fluid, passing through the side wall (11b) of said solidification cup (11)
- and auxiliary ventilation means (14a).

5. Apparatus (10) according to one or more of the preceding claims, **characterised in that** said protection ferrule (15) comprises a conical body (15a), for lateral protection, and an upper cap (15b), configured to contact the tip of said central thermocouple (16).

6. Apparatus (10) according to one or more of the preceding claims, **characterized in that** it comprises one or more side thermocouples (22), each inserted into a corresponding seat (23) defined in the side wall (11b) of the solidification cup (11), so that the tip of the side thermocouple (22) is close to the inner surface of the containment compartment (12).

7. Apparatus (10) according to one or more of the preceding claims, **characterised in that** it comprises a ladle (50) for transporting molten metallic material, said ladle (50) comprising a container having at least two layers, said ladle (50) being configured to reduce heat losses during the transfer of the molten metallic material.

8. Method of operation of an apparatus (10) according to one or more of the preceding claims, **characterised by** comprising the following operating steps:
- pre-heating of said solidification cup (11) up to a predefined temperature (Ti);
- pouring of a non-ferrous alloy in the molten state into said containment compartment (12) of said solidification cup (11);
- controlled cooling of said non-ferrous alloy by said heating means (13) and by said cooling means (14);
- measurement of temperature variations of said non-ferrous alloy by said central thermocouple (16);
- ejection of the solidified sample of said non-ferrous alloy from said containment compartment (12);
- measurement of the shrinkage percentage of said sample of solidified non-ferrous metallic material by said measuring device (60).

9. Method according to the preceding claim, **characterized in that** said pouring step is carried out with said ladle (50), said ladle (50) being pre-heated by positioning it in the molten non-ferrous metallic material prior to the withdrawal of the molten non-ferrous metallic material itself.

## Patentansprüche

1. Einrichtung (10) zur Analyse von Nichteisenmetall-Legierungen, insbesondere für Analysen vom Typ "STA", umfassend:
- einen Erstarrungsbecher (11), in dem ein Aufnahmeraum (12) zur Aufnahme eines Metallmaterials im geschmolzenen Zustand definiert ist;
- Heizmittel (13), die dazu konfiguriert sind, den Erstarrungsbecher (11) zu erwärmen, und die so angeordnet sind, dass sie den Aufnahmeraum (12) umgeben;
- Kühlmittel (14), die dazu konfiguriert sind, den Erstarrungsbecher (11) zu kühlen;
- eine Schutzhülse (15), die in das Innere des Aufnahmeraums (12) hineinragt und dazu konfiguriert ist, das Ende eines Thermoelements aufzunehmen und zu bedecken;
- ein mittiges Thermoelement (16), das in eine entsprechende Durchgangsbohrung (17) eingesetzt ist, die im Boden (11a) des Erstarrungsbechers (11) definiert ist, wobei das Ende des mittigen Thermoelements (16) in der Schutzhülse (15) positioniert ist;
- Arretierungsmittel (18), die dazu konfiguriert sind, das mittige Thermoelement (16) stabil in seiner Position zu arretieren;
- Auswurfmittel (19), die dazu konfiguriert sind, den Auswurf eines Körpers aus erstarrtem Metallmaterial aus dem Aufnahmeraum (12) ermöglichen;
- ein Thermoelement des Bechers (24) zum Messen der Temperatur des Erstarrungsbechers (11);
- eine elektronische Steuereinheit (150), die dazu konfiguriert ist, die Heizmittel (13) und die Kühlmittel (14) ein- und auszuschalten und Signale von dem mittigen Thermoelement (16) und von dem Thermoelement des Bechers (24) zu empfangen und zu senden, **dadurch gekennzeichnet, dass** sie
- eine Vorrichtung (60) zum Messen der Schrumpfung, in Prozent, der erstarrten Proben umfasst.

2. Einrichtung (10) nach Anspruch 1, **dadurch gekennzeichnet, dass** sie einen herausnehmbaren, trinkglasförmigen Tiegel (125) umfasst, der so geformt ist, dass er in den Aufnahmeraum (12) eingesetzt werden kann, und der herausnehmbar in denselben Aufnahmeraum (12) eingesetzt ist, wobei der herausnehmbare, trinkglasförmige Tiegel (125) eine mittige Erhebung (126) aufweist, die so konfiguriert ist, dass sie das mittige Thermoelement (16) aufnimmt.

3. Einrichtung (10) nach Anspruch 1, **dadurch gekennzeichnet, dass** die Heizmittel (13) vom Induktionstyp oder alternativ vom Widerstandstyp (413) sind.

4. Einrichtung (10) nach einem oder mehreren der vorstehenden Ansprüche, **dadurch gekennzeichnet, dass** die Kühlmittel (14) mindestens eines umfassen, ausgewählt aus:
- einer Zirkulationswindung (11e, 11f) für eine Kühlflüssigkeit, die durch die Seitenwand (11b) des Erstarrungsbechers (11) verläuft
- und zusätzlichen Ventilationsmitteln (14a).

5. Einrichtung (10) nach einem oder mehreren der vorstehenden Ansprüche, **dadurch gekennzeichnet, dass** die Schutzhülse (15) einen konischen Körper (15a), zum seitlichen Schutz, und eine obere Kappe (15b), die so konfiguriert ist, dass sie die Spitze des mittigen Thermoelements (16) berührt, umfasst.

6. Einrichtung (10) nach einem oder mehreren der vorstehenden Ansprüche, **dadurch gekennzeichnet, dass** sie ein oder mehrere seitliche Thermoelemente (22) umfasst, die jeweils in einen entsprechenden Sitz (23) eingesetzt sind, der in der Seitenwand (11b) des Erstarrungsbechers (11) derart definiert ist, dass die Spitze des seitlichen Thermoelements (22) nahe der Innenfläche des Aufnahmeraums (12) liegt.

7. Einrichtung (10) nach einem oder mehreren der vorstehenden Ansprüche, **dadurch gekennzeichnet, dass** sie eine Pfanne (50) zum Transportieren von geschmolzenem Metallmaterial umfasst, wobei die Pfanne (50) einen Behälter mit mindestens zwei Schichten umfasst und die Pfanne (50) so konfiguriert ist, dass Wärmeverluste während der Übertragung des geschmolzenen Metallmaterials verringert werden.

8. Verfahren zum Betreiben einer Einrichtung (10) nach einem oder mehreren der vorstehenden Ansprüche, **gekennzeichnet durch** das Umfassen der folgenden Arbeitsschritte:
- Vorheizen des Erstarrungsbechers (11) auf eine vorgegebene Temperatur (Ti);
- Gießen einer Nichteisenlegierung im geschmolzenen Zustand in den Aufnahmeraum (12) des Erstarrungsbechers (11);
- kontrolliertes Abkühlen der Nichteisenlegierung durch die Heizmittel (13) und durch die Kühlmittel (14);
- Messen der Temperaturänderungen der Nichteisenlegierung durch das mittige Thermoelement (16);
- Auswerfen der erstarrten Probe der Nichteisenlegierung aus dem Aufnahmeraum (12);
- Messen der Schrumpfung, in Prozent, der Probe aus erstarrtem Nichteisenmetallmaterial durch die Messvorrichtung (60).

9. Verfahren nach dem vorstehenden Anspruch,
**dadurch gekennzeichnet, dass** der Gießschritt mit der Pfanne (50) durchgeführt wird, wobei die Pfanne (50) vorgewärmt wird, indem sie in das geschmolzene Nichteisenmetallmaterial gestellt wird, bevor das geschmolzene Nichteisenmetallmaterial an sich entnommen wird.

## Revendications

1. Appareil (10) pour l'analyse d'alliages métalliques non ferreux, en particulier pour des analyses de type "S.T.A.", comprenant :
- une coupelle de solidification (11) à l'intérieur de laquelle est défini un compartiment de confinement (12) destiné à recevoir un matériau métallique à l'état fondu ;
- des moyens de chauffage (13), conçus pour chauffer ladite coupelle de solidification (11), positionnés de manière à entourer ledit compartiment de confinement (12) ;
- des moyens de refroidissement (14), conçus pour refroidir la coupelle de solidification (11) ;
- une virole de protection (15), faisant saillie à l'intérieur dudit compartiment de confinement (12), conçue pour recevoir et couvrir l'extrémité d'un thermocouple ;
- un thermocouple central (16), inséré dans un trou de passage correspondant (17) défini au fond (11a) de ladite coupelle de solidification (11), l'extrémité dudit thermocouple central (16) étant positionnée dans ladite virole de protection (15) ;
- un moyen de verrouillage (18) conçu pour verrouiller de manière stable ledit thermocouple central (16) dans sa position ;
- un moyen d'éjection (19) conçu pour permettre l'éjection d'un corps de matériau métallique solidifié hors dudit compartiment de confinement (12) ;
- un thermocouple de la coupelle (24) pour mesurer la température de ladite coupelle de solidification (11) ;
- une unité de commande électronique (150) configurée pour activer et désactiver lesdits moyens de chauffage (13) et lesdits moyens de refroidissement (14), et pour recevoir et émettre des signaux à partir dudit thermocouple central (16) et dudit thermocouple de la coupelle (24), **caractérisé en ce qu'**il comprend ;
- un dispositif (60) pour mesurer le pourcentage de rétrécissement des échantillons solidifiés.

2. Appareil (10) selon la revendication 1, **caractérisé en ce qu'**il comprend un creuset amovible en forme de verre (125) formé pour être inséré dans le compartiment de confinement (12), et inséré de manière amovible dans le même compartiment de confinement (12), ledit creuset amovible en forme de verre (125) ayant un relief central (126) conçu pour recevoir ledit thermocouple central (16).

3. Appareil (10) selon la revendication 1, **caractérisé en ce que** lesdits moyens de chauffage (13) sont du type à induction, ou alternativement du type résistif (413).

4. Appareil (10) selon l'une ou plusieurs des revendications précédentes, **caractérisé en ce que** lesdits moyens de refroidissement (14) comprennent au moins un élément choisi parmi :
- un serpentin de circulation (11e, 11f) pour un fluide de refroidissement, traversant la paroi latérale (11 b) de ladite coupelle de solidification (11)
- et des moyens de ventilation auxiliaires (14a).

5. Appareil (10) selon l'une ou plusieurs des revendications précédentes, **caractérisé en ce que** ladite virole de protection (15) comprend un corps conique (15a), pour une protection latérale, et un capuchon supérieur (15b), conçu pour entrer en contact avec la pointe dudit thermocouple central (16).

6. Appareil (10) selon l'une ou plusieurs des revendications précédentes, **caractérisé en ce qu'**il comprend un ou plusieurs thermocouples latéraux (22), chacun inséré dans un siège correspondant (23) défini dans la paroi latérale (11b) de la coupelle de solidification (11), de sorte que la pointe du thermocouple latéral (22) est proche de la surface interne du compartiment de confinement (12).

7. Appareil (10) selon l'une ou plusieurs des revendications précédentes, **caractérisé en ce qu'**il comprend une poche (50) pour le transport d'un matériau métallique fondu, ladite poche (50) comprenant un récipient ayant au moins deux couches, ladite poche (50) étant conçue pour réduire les pertes de chaleur lors du transfert du matériau métallique fondu.

8. Procédé de fonctionnement d'un appareil (10) selon l'une ou plusieurs des revendications précédentes, **caractérisé en ce qu'**il comprend les étapes de fonctionnement suivantes :
- le préchauffage de ladite coupelle de solidification (11) jusqu'à une température prédéfinie (Ti) ;
- la coulée d'un alliage non ferreux à l'état fondu dans ledit compartiment de confinement (12) de ladite coupelle de solidification (11) ;
- le refroidissement commandé dudit alliage non ferreux par lesdits moyens de chauffage (13) et par lesdits moyens de refroidissement (14) ;
- la mesure des variations de température dudit alliage non ferreux par ledit thermocouple central (16) ;
- l'éjection de l'échantillon solidifié dudit alliage non ferreux hors dudit compartiment de confinement (12) ;
- la mesure du pourcentage de rétrécissement dudit échantillon de matériau métallique non ferreux solidifié par ledit dispositif de mesure (60).

9. Procédé selon la revendication précédente, **caractérisé en ce que** ladite étape de coulée est effectuée avec ladite poche (50), ladite poche (50) étant préchauffée en la positionnant dans le matériau métallique non ferreux fondu avant le retrait du matériau métallique non ferreux fondu lui-même.
